(19) Europäisches Patentamt
European Patent Office
Office européen des brevets

(11) EP 1 729 773 B1

(12) **EUROPEAN PATENT SPECIFICATION**

(45) Date of publication and mention of the grant of the patent:
**02.07.2008 Bulletin 2008/27**

(21) Application number: **05730069.1**

(22) Date of filing: **24.03.2005**

(51) Int Cl.:
***A61K 31/47*** *(2006.01)* ***C07D 207/48*** *(2006.01)*
***C07D 215/26*** *(2006.01)* ***C07D 215/60*** *(2006.01)*

(86) International application number:
**PCT/EP2005/003144**

(87) International publication number:
**WO 2005/089760 (29.09.2005 Gazette 2005/39)**

(54) **8-HYDROXY-5- [(-HYDROXY-2- [[ (1R)-2-(4-METHOXYPHENYL)-1-METHYLETHYL] AMINO ] [ETHYL -2(1H)-QUINOLINONE MONOHYDROCHLORIDE IN CRYSTALLINE FORM AND THE PROCESS FOR ITS PREPARATION**

8-HYDROXY-5- [(-HYDROXY-2- [[ (1R)-2-(4-METHOXYPHENYL)-1-METHYLETHYL] AMINO ] [ETHYL -2(1H)-CHINOLINONMONOHYDROCHLORID IN KRISTALLINER FORM UND HERSTELLUNGSVERFAHREN DAFÜR

MONOHYDROCHLORURE DE 8-HYDROXY-5-[(1R)-1-HYDROXY-2-[[(1R)-2-(4-METHOXYPHENYL)-1-METHYLETHYL]AMINO]ETHYL]-2(1H)-QUINOLINONE SOUS FORME CRISTALLINE ET PROCEDE DE PREPARATION ASSOCIE

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **24.03.2004 EP 04007045**

(43) Date of publication of application:
**13.12.2006 Bulletin 2006/50**

(73) Proprietor: **CHIESI FARMACEUTICI S.p.A.**
**I-43100 Parma (IT)**

(72) Inventors:
- **PIVETTI, Fausto**
  **I-43100 Parma (IT)**
- **PIGHI, Roberto**
  **I-43100 Parma (IT)**

(74) Representative: **Minoja, Fabrizio**
**Bianchetti Bracco Minoja S.r.l.**
**Via Plinio, 63**
**20129 Milano (IT)**

(56) References cited:
**WO-A-20/05013945** **US-A- 4 579 854**
**US-A- 6 030 604**

- **KIKKAWA H ET AL: "TRACHEAL RELAXING EFFECTS AND BETA2-SELECTIVITY OF TA-2005, A NEWLY DEVELOPED BRONCHODILATING AGENT, IN ISOLATED GUINEA PIG TISSUES" JAPANESE JOURNAL OF PHARMACOLOGY, THE JAPANESE PHARMACOLOGICAL SOCIETY, KYOTO, JP, vol. 57, no. 2, 1991, pages 175-185, XP008045059 ISSN: 0021-5198**
- **VOSS, HANS PETER ET AL: "Atypical molecular pharmacology of a new long-acting .beta.2-adrenoceptor agonist, TA 2005" EUROPEAN JOURNAL OF PHARMACOLOGY, MOLECULAR PHARMACOLOGY SECTION , 227(4), 403-9 CODEN: EJPPET; ISSN: 0922-4106, 1992, XP002334109**
- **DATABASE CA [Online] CHEMICAL ABSTRACTS SERVICE, COLUMBUS, OHIO, US; HOSHIKO, KENICHIRO ET AL: "Treatment of inflammatory diseases with drugs containing carbostyril derivative" XP002334111 retrieved from STN Database accession no. 1997:769190 -& JP 09 309830 A (NOVARTIS A. -G., SWITZ.) 2 December 1997 (1997-12-02)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 729 773 B1

- DATABASE CA [Online] CHEMICAL ABSTRACTS SERVICE, COLUMBUS, OHIO, US; IZEBOUD, C. A. ET AL: "Stereoselectivity at the .beta.2-adrenoceptor on macrophages is a major determinant of the anti-inflammatory effects of .beta.2-agonists" XP002334112 retrieved from STN Database accession no. 2000:627207 & NAUNYN-SCHMIEDEBERG'S ARCHIVES OF PHARMACOLOGY , 362(2), 184-189 CODEN: NSAPCC; ISSN: 0028-1298, 2000,
- ROSSONI, GIUSEPPE ET AL: "Positive interaction of the .beta.2-agonist CHF 4226.01 with budesonide in the control of bronchoconstriction induced by acetaldehyde in the guinea-pigs" BRITISH JOURNAL OF PHARMACOLOGY , 144(3), 422-429 CODEN: BJPCBM; ISSN: 0007-1188, 10 January 2005 (2005-01-10), XP002334110

## Description

The present invention relates to 8-hydroxy-5-[(1R)- 1- hydroxy- 2-[[(1R)- 2-(4- methoxyphenyl)- 1-methylethyl]amino]ethyl]-2(1H)-quinolinone monohydrochloride of formula (I):

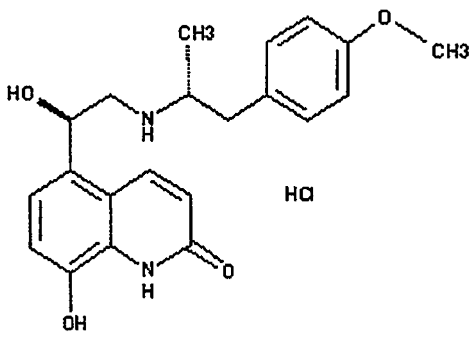

(I)

in crystalline form. The invention also relates to the process for the isolation by crystallisation of the compound (I) and to its use for the preparation of pharmaceutical compositions for inhalation in combination with suitable carriers or vehicles.

## Background of the invention

8- hydroxy- 5-[(1R)- 1- hydroxy- 2-[[(1R)- 2-(4-methoxyphenyl)-1-methylethyl]amino]ethyl]2(1H)-quinolinone monohydrochloride is known from the European patent n. EP 0 147 719 as a bronchodilator provided with a potent beta-2-adrenoceptor stimulating action.

The compound, that has been also defined as 8-hydroxy-5-{(1R)-1-hydroxy-2-[N-((1R)-2-(p-methoxyphenyl)-1-methylethyl)amino]ethyl} carbostyril hydrochloride and referred to as TA 2005, has been further developed by the applicant under the experimental code CHF 4226.

## Prior art

The process for the preparation of TA 2005 is described in EP 0 147 719, example 4. In particular, the process for the isolation of the crude product is reported in step (3-a), wherein the insoluble materials obtained after the catalytic hydrogenation of 3.5 g of 8-benzyloxy-5-{(1R)-1-hydroxy-2-[N-((1R)-2-(p-methoxyphenyl)-1-methylethyl)amino]ethyl}carbostyril hydrochloride in tetrahydrofuran (100 ml) and water (10 ml) are collected by filtration and washed with an aqueous 10% ethanol solution. The filtrate and washings are combined, and the combined solution is concentrated under reduced pressure to remove solvent. The residue is crystallised with a mixture of ethanol, water and isopropyl ether, and crystalline precipitates are collected by filtration. 2.38 g of 8-hydroxy-5-{(1R)-1-hydroxy-2-[N-((1R)-2-(p-methoxyphenyl)-1-methylethyl)amino]ethyl}carbostyril hydrochloride are obtained as colorless crystals.

The yield was of 83% and the final product showed the following characteristics:
melting point: 170.0-171.5°C (decomp.)

$[\alpha]_D^{22}$ -64.40° (c= 1.00, methanol)

IR $\nu_{max}^{mujol}$ (cm$^{-1}$): 3300 (broad), 1640, 1610, 1600

JP 09-309830 discloses the use of TA 2005 or salts thereof for the prevention or treatment of inflammatory states (eosinophilia, allergy, asthma, dermatitis, rhinitis, etc.); the product may be in the form of topical preparations, inhalants, transdermal or pernasal preparations.

US 6,030,604 discloses powder formulations for inhalation which can comprise different kinds of active ingredients, among which TA 2005 is generically cited.

Kikkawa H et al in Japan J Pharmacol 57, 175-185, 1991; Voss HP et al in Eur J Pharmacol - Mol Pharmacol Sect 227, 403-409, 1992; Izeboud CA et al in Naunyn-Schmiedeberg's Archv Pharmacol 362 (2), 184-189, 2000 describe the in vitro pharmacological effects of TA 2005. In particular, the latter describes the differences between the pharmacological activity of the two stereoisomer forms of TA 2005: the (R,R) form, also known as TA 2005 and the (S,S) form.

J Pharmacol 144, 422-429, 2005 describes the positive interaction of tracheal instillation of CHF 4226.01 (TA 2005) and budesonide in the control of bronchoconstriction induced by acetaldehyde in the guinea-pigs.

WO2005/013945 discloses pharmaceutical compositions comprising one or more steroids and a beta-2 agonist of formula corresponding to TA 2005, diastereoisomers, mixture of diastereoisomers or racemates thereof.

## Object of the invention

The invention relates to 8-hydroxy-5-[(1R)-1-hydroxy- 2-[[(1R)- 2-(4- methoxyphenyl)- 1- methylethyl] amino]ethyl]-2(1H)-quinolinone monohydrochloride in crystalline form having suitable characteristics for the preparation of pharmaceutical compositions for inhalation in combination with suitable carriers or vehicles.

The compound will be identified hereinafter for sake of brevity also with the code CHF 4226.

The compound of the invention is preferably administered by inhalation.

Formulations for inhalation wherein the active compound is in solid form include dry powder compositions to be delivered by a dry powder inhaler (DPI), aerosol compositions comprising a suspension of fine drug particles in a propellant gas to be delivered by a pressurized metered-dose inhaler (pMDI) and aerosol composition in form of aqueous suspensions to be delivered by a nebulizer.

The efficacy of this route of administration can be limited by the problem encountered in making appropriate and consistent dosages available to the lungs.

One of the most important features is to ensure uniform distribution of the active compound in the formulation, particularly when it is highly potent and has to be given in low doses.

Moreover, the solid compound in the composition should be as pure as possible and endowed with the required chemical and physical stability.

In addition, in the compositions for inhalation, the active compound at the solid state should be present in the form of finely divided particles of a controlled particle size which does not exceed approximately a mass median diameter (MMD) of 10 μm, preferably 6 μm, more preferably 5 μm, in order to achieve maximum penetration into the lungs.

Said particles are conventionally prepared by techniques such as micronization or grinding.

Such techniques can produce particles which have regions of partially amorphous structure and are liable to change their structure when kept in various environmental conditions and/or processed for the preparation of pharmaceutical compositions.

Therefore, the particles of the active compound should be provided with an adequate degree of crystallinity in order to be highly stable during the grinding or micronization process and sufficiently stable for the subsequent pharmaceutical use.

The aim of the present invention is thus to provide a stable crystalline 8-hydroxy-5-[(1R)-1-hydroxy-2-[[(1R)-2-(4-methoxyphenyl)-1-methylethyl] amino] ethyl]-2(1H)-quinolinone monohydrochloride.

Another aim of the invention is to provide a process for the preparation of the compound with an adequate degree of crystallinity. The compound of the invention is chemically and physically stable and maintains the same degree of crystallinity, also after having undergone micronization or grinding processes.

In the prior art, in order to obtain 8-hydroxy-5-{(1R)- 1- hydroxy- 2-[N-((1R)- 2-(p- methoxyphenyl)- 1-methylethyl)amino]ethyl} carbostyril hydrochloride, after collecting the crude product and washing with an aqueous 10% ethanol solution, the filtrate and washings were combined and the combined solution was concentrated under reduced pressure to remove the solvent, the residue was crystallised with a mixture of ethanol, water and isopropyl ether, and crystalline precipitates were collected by filtration, but no teaching was given on how to perform the crystallisation process.

It has now been found that in said process of crystallisation, wherein the residue obtained after removing the solvent is dissolved by heating in an ethanol water mixture (95:5) and the solution is concentrated under reduced pressure to remove part of the solvent, the volume to which the solution is reduced is critical. It has indeed been found that the solution should be concentrated to a volume equal to or higher than 1/3 of its initial volume. Moreover the isopropyl ether has to be added to the concentrated solution slowly, in not less than 5 minutes and at a temperature higher than 30°C.

The above specified conditions allow to obtain an homogeneous solution wherein a uniform and regular crystalline growth takes place.

In fact it has been found that if the volume of the concentrate of the crude product is too small and in particular smaller than 1/3 of its initial volume and the addition of isopropyl ether is effected too fastly, for example in less than 5 minutes, the highly concentrated crude compound rapidly precipitates, thick unfilterable slurries are formed, the compound incorporates high levels of mother liquors consisting of solvents and impurities dissolved therein and it can be isolated very hardly. Moreover, when isolated and dried, it includes a significant amount of impurities.

Furthermore, a high percent amount of compound in amorphous state is present and, as highlighted before, particles of amorphous structure can cause a number of problems when included in inhalation formulations: in fact this kind of particles are extremely cohesive, tend to sti ck together and tend to absorb ambient moisture at their surfaces during the time.

So there is the need for a compound with an adequate degree of purity and an adequate degree of crystallinity.

The present invention provides CHF 4226 in a pure crystalline form and a process for preparation thereof.

In order to prepare the crystalline compound of the invention, crude 8-hydroxy-5-[(1R)-1-hydroxy-2-[[(1R)-2-(4-methoxyphenyl)-1-methylethyl] amino] ethyl]-2(1H)-quinolinone monohydrochloride which has been obtained for example according to the method disclosed in EP 0 147 719 by crystallisation from a mixture of ethanol, water and isopropyl ether is recrystallised from a suitable solvent.

A suitable recrystallisation solvent is a protic solvent such as ethanol, isopropanol or their aqueous mixtures. The preferred solvent is an ethanol-water mixture.

The most suitable recrystallisation solvent is an ethanol-water mixture in a ratio from 97:3 to 95:5 v/v. Advantageously, after the dissolution of the crude compound in the above mentioned solvent and before the isolation of the final product, an intermediate step of distillation of the aqueous ethanolic solution under reduced pressure is carried out, to remove residual isopropyl ether from the mixture as well as to improve the yield.

Preferably the distillation process is continued until the solution is reduced to a volume comprised between ½ and 1/3 of the initial volume.

The recrystallisation process according to the invention allows an effective removal of the impurities up to levels equal to or lower than 0.5%, preferably 0.2%,

even more preferably 0.1% in order to obtain the compound in a pure crystalline form provided with suitable characteristics to be used for the preparation of pharmaceutical compositions for inhalation in combination with suitable carriers or vehicles.

**Detailed description of the invention**

The method of EP 0 147 719, whose teaching is incorporated in full in the present application, involves the filtration and washing with aqueous ethanol of the suspension obtained after the catalytic hydrogenation of 8-benzyloxy-5-{(1R)-1-hydroxy-2-[N-((1R)-2-(p-methoxyphenyl)-1-methylethyl)amino]ethyl}carbostyril hydrochloride to remove the catalyst. The solution is concentrated under reduced pressure to remove the solvent. According to the present invention the residue obtained after removing the solvent is dissolved by heating in an ethanol water mixture in the preferred ratio of 95:5 and the solution so obtained is concentrated under reduced pressure, preferably comprised between 200 and 400 mbar, at a temperature comprised between 30°C and 55°C, preferably at a temperature from 45 to50°C, to a volume comprised between 1/2 and 1/3 of the initial volume. Then diisopropyl ether is slowly added to the warm solution under stirring. The addition of diisopropyl ether is performed in at least 5 minutes, preferably in more than 10 minutes and more preferably in an interval from 20 to 30 minutes.

The mixture is then cooled under stirring at a temperature be-tween 0°C and 10°C for 1 to 2 hours and the solid is isolated and washed with ethanol.

The wet crude product is suspended in ethanol, heated under reflux at 75-78°C and slowly added with water until a clear solution is obtained. The solution is filtered and the filter is washed with ethanol. The warm solution is concentrated, under stirring, under reduced pressure, at a temperature not lower than 40°C, preferably comprised between 40 and 50°C, more preferably comprised between 45 and 48°C, to a volume ranging from about 1/2 to about 1/3 of its starting volume. The product begins to crystallise from the solution giving rise to a suspension. The suspension is slowly cooled and kept at a temperature from about 0 to 10°C, preferably from about 0 to 5°C, for at least 1 hour and up to 20 hours or more, under stirring. The solid is recovered by filtration, washed with ethanol and finally dried in a conventional manner, for example by air drying, drying under reduced pressure, or drying in the presence of a sterile inert gas to give the crystalline compound.

The 8-hydroxy-5-[(1R)-1-hydroxy-2-[[(1R)-2-(4- methoxyphenyl)- 1- methylethyl] amino] ethyl]- 2 (1H)-quinolinone monohydrochloride obtainable using the method described above was investigated to determine: the melting point by Differential Scanning Calorimetry (DSC), the specific optical rotatory power $[\alpha]_D^{20}$, the enantiomeric purity by capillary zone electrophoresis and by High Performance Liquid Chromatography (HPLC), the amount of total impurity by HPLC and the X-ray powder diffraction (XRD) pattern.

The 8-hydroxy-5-[(1R)-1-hydroxy-2-[[(1R)-2-(4- methoxyphenyl)- 1- methylethyl] amino] ethyl]- 2 (1H)-quinolinone monohydrochloride of the present invention is characterized by:

- a melting range of 180°-200°C (with decomposition), pre ferably of 185-195°C (with decomposition), more preferably of 190-195°C (with decomposition) determined by DSC at a scan rate of 10°C/min.;
- a specific optical rotatory power $[\alpha]_D^{20}$ (c = 1.00, methanol) of -68.0°;
- an enantiomeric purity higher than 99.0%, preferably higher than 99.5%, determined by capillary zone electrophoresis and by HPLC;
- impurity levels of less than 0.5%, preferably less than 0.2%, even more preferably less than 0.1 %, determined by HPLC;
- a X-ray powder diffraction pattern identical or substantially identical to that listed under the example below. Suitably the compound has *inter alia* one or more of the following characteristic XRD peaks: 12.2; 13.6; 16.3; 18.0; 18.2; 19.2; 21.4; 21.9; 22.8; 23.5; 24.2; 24.9; 26.6; 28.5; 29.4; 29.9; and 33.9 $\pm$ 0.2 degrees /2 theta;
- a crystalline degree, expressed as weight % of the crystalline compound with respect to the total weight of the compound, of at least 90%, preferably of at least 93%, even more preferably of at least 95%, determined according to a microcalorimetric method developed in-house.

The following example illustrates the present invention.

**EXAMPLE**

Crystallisation of 8-hydroxy-5-[(1R)-1-hydroxy-2-[[(1R)- 2-(4- methoxyphenyl)- 1- methylethyl] amino] ethyl]-2(1H)-quinolinone monohydrochloride (CHF 4226 monohydrochloride)

In order to prepare a crystalline CHF 4226 according to the present invention, the residue which has been obtained, for example, after the catalytic hydrogenation of 8-benzyloxy-5-{(1R)-1-hydroxy-2-[N-((1R)-2-(p-methoxy phenyl)-1-methylethyl) amino]ethyl}carbostyril hydrochloride (100 g) as described in the step 3-a of Example 4 of EP 0 147 719 was dissolved in about 1300 ml of ethanol and 100 ml of water and concentrated in a rotary evaporator (Tbath = 55°C; vacuum = - 0.8 bar) until the residual volume was about 600 ml. Isopropyl ether (560 ml) was dropped in the warm solution (T = 45-50°C) in 30 min. The mixture was cooled at 5-10°C and stirred for 60 minutes until the crystallization process is completed, then it was filtered in a Buckner filter washing the solid with 200 ml of ethanol.

The wet crude 8-hydroxy-5-[(1R)-1-hydroxy-2-[[(1R)-2-(4-methoxy phenyl)-1-methylethyl]amino] ethyl]-2(1H)-quinolinone monohydrochloride (154.6 g; corresponding to 73 g if dried at 60°C under vacuum) was suspended in ethanol (580 ml) and the suspension was heated at 78°C under reflux; 25 ml of water were slowly dropped in, until a clear solution was obtained. The hot solution was filtered in a Buckner filter, washing with 150 ml of ethanol. The filtered solution was concentrated under vacuum (Tbath = 55-65°C; vacuum = 250-300 mbar; Tsolution = 45-48°C), distilling about 360 ml of solvent. Vacuum was disconnected and 390 ml of ethanol were added to the residual suspension, stirring at 45°C until an homogeneous suspension was obtained. The suspension was cooled at a temperature lower than 5°C in 90 min., then it was kept at 5°C for 20 hours. The suspension was filtered in a Buckner filter, washing with 150 ml of ethanol. The solid was then dried under vacuum at 60°C for 24 hours. 58.4 g of 8-hydroxy-5-[(1R)-1-hydroxy-2-[[(1R)-2-(4-methoxyphenyl)-1-methylethyl]amino]ethyl]-2(1H)-quinolinone monohydrochloride were obtained (80.0% yield), having the following characteristics:

- melting range 190-194°C (decomposition), determined by DSC at a scan rate of 10°C/min.;
- specific optical rotatory power $[\alpha]_D^{20}$ (c = 1.00, methanol) = -68.0°;
- enantiomeric purity higher than 99.5%, determined by capillary zone electrophoresis;
- total impurities: less than 0.1%;
- the X-ray powder diffraction (XRD) pattern is shown in Figure 1 and is represented by the following major peaks:

| Angle [°/2θ] | Rel. Int. [%] |
|---|---|
| 12.3 | 21.0 |
| 13.6 | 54.2 |
| 16.4 | 64.9 |
| 18.0 | 37.5 |
| 18.4 | 44.5 |
| 19.3 | 50.1 |
| 21.4 | 55.8 |
| 21.9 | 100.0 |
| 22.9 | 35.6 |
| 23.6 | 36.9 |
| 24.3 | 88.4 |
| 25.0 | 21.4 |
| 26.7 | 32.5 |
| 28.6 | 22.6 |
| 29.5 | 30.5 |
| 29.9 | 14.8 |
| 34.0 | 20.4 |

The crystalline degree of the compound has been determined according to a differential scanning calorimetry (DSC) method developed in-house based on the measurement of the heat of fusion.

Said method uses a scan rate of 130° C/min to evaluate the percentage of crystalline compound in a sample by determining the ratio between the ΔH (heat of fusion) of the sample with respect to the ΔH of a 100% crystalline reference standard, determined in the same range of temperature and in the same experimental conditions. The 100% crystalline reference standard was prepared by suspending the compound in ethanol, then filtering to eliminate the residual amorphous compound dissolved in ethanol, and drying.

The method was applied to samples of the compound of the Example as such, and after grinding or micronization. All the samples showed a cristallinity degree higher than 90% which was maintained also after subjecting the compound to processes of grinding and micronization.

## Claims

1. A process for the preparation of 8-hydroxy-5-[(1R)-1-hydroxy-2-[[(1R)-2-(4-methoxyphenyl)-1-methylethyl]aminoJethyl]-2(1H)-quinolinone mono-hydrochloride (I) comprising crystallising or re-crystallising compound (I) from an ethanol-water mixture in a ratio from 97:3 to 95:5 added with diisopropyl ether, wherein the ethanol-water mixture is concentrated under reduced pressure at a temperature comprised between 30 and 55° C to a volume comprised between 1/2 and 1/3 of the initial volume and the addition of the diisopropyl ether is performed in at least 5 minutes.

2. A process according to claim 1 further comprising the step of recrystallization from a protic solvent comprising ethanol, isopropanol or their aqueous mixtures.

3. 8-Hydroxy-5-[(1R)-1-hydroxy-2-[[(1R)-2-(4-methoxyphenyl)-1-methylethyl] amino] ethyl]-2 (1H)-quinolinone monohydrochloride obtainable by the process according to any one of claims 1-2 having a crystalline degree expressed as weight % of the crystalline compound with respect to the total weight of the compound of at least 93%.

4. 8-Hydroxy-5-[(1R)-1-hydroxy-2-[[(1R)-2-(4-methoxyphenyl)-1-methylethyl] amino]ethyl]-2(1H)-quinolinone monohydrochloride according to claim 3 having a crystalline degree expressed as weight % of the crystalline compound with respect to the total weight of the compound of at least 95%.

5. 8-hydroxy-5-[(1R)-1-hydroxy-2-[[(1R)-2-(4-methoxyphenyl)-1-methylethyl] amino] ethyl]-2(1H)-quinolinone monohydrochloride according to any one of claims 3-4 having a melting range of 185-195°C determined by Differential Scanning Calorimetry,

6. 8-hydroxy-5-[(1R)-1-hydroxy-2-[[(1R)-2-(4-methoxyphenyl)-1-methylethyl] amino] ethyl]-2(1H)-quinolinone monohydrochloride according to any one of claims 3-4 having a melting range of 190-195°C determined by Differential Scanning Calorimetry.

## Patentansprüche

1. Verfahren zur Herstellung von 8-Hydroxy-5-[(1R)-1-hydroxy-2-[[(1R)-2-(4-methoxyphenyl)-1-methylethyl] amino]-ethyl]-2 (1H)-chinolinon-Monohydrochlorid (I), umfassend Kristallisieren oder Umkristallisieren von Verbindung (I) aus einem Ethanol-Wasser-Gemisch in einem Verhältnis von 97:3 bis 95:5, versetzt mit Diisopropylether, wobei das Ethanol-Wasser-Gemisch unter reduziertem Druck bei einer Temperatur zwischen 30 und 55°C auf ein Volumen konzentriert wird, das zwischen 1/2 und 1/3 des Anfangsvolumens ausmacht, und wobei die Zugabe des Isopropylethers in wenigstens 5 Minuten durchgeführt wird.

2. Verfahren nach Anspruch 1, das außerdem den Schritt des Umkristallisierens aus einem protischen Lösungsmittel, das Ethanol, Isopropanol oder deren wässrige Gemische umfasst, umfasst.

3. 8- Hydroxy- 5-[(1R)- 1- hydroxy- 2-[[(1R)- 2-(4- methoxyphenyl)-1-methylethyl]amino]ethyl]-2(1H)-chinolinon-Monohydrochlorid, erhältlich durch das Verfahren nach einem der Ansprüche 1-2, das einen kristallinen Grad, ausgedrückt als Gewichtsprozent der kristallinen Verbindung, bezogen auf das Gesamtgewicht der Verbindung, von wenigstens 93% hat.

4. 8- Hydroxy- 5-[(1R)- 1- hydroxy- 2-[[(1R)- 2-(4- methoxyphenyl)-1-methylethyl]amino]ethyl]-2(1H)-chinolinon-Monohydrochlorid nach Anspruch 3 mit einem kristallinen Grad, ausgedrückt als Gewichtsprozent der kristallinen Verbindung, bezogen auf das Gesamtgewicht der Verbindung, von wenigstens 95%.

5. 8- Hydroxy- 5-[(1R)- 1- hydroxy- 2-[[(1R)- 2-(4- methoxyphenyl)-1-methylethyl]amino]ethyl]-2(1H)-chinolinon-Monohydrochlorid nach einem der Ansprüche 3-4 mit einem Schmelzbereich von 185-195°C, bestimmt durch Differenzialscanningkalorimetrie.

6. 8- Hydroxy- 5-[(1R)- 1- hydroxy- 2-[[(1R)- 2-(4- methoxyphenyl)-1-methylethyl]amino]ethyl]-2(1H)-chinolinon-Monohydrochlorid nach einem der Ansprüche 3-4 mit einem Schmelzbereich von 190-195°C, bestimmt durch Differenzialscanningkalorimetrie.

## Revendications

1. Procédé pour la préparation de monochlorhydrate de 8-hydroxy-5-[(1R)-1-hydroxy-2-[[(1R)-2-(4-méthoxyphényl)-1-méthyléthyl]amino]éthyl]-2(1H)-quinolinone (I) comprenant la cristallisation ou la recristallisation du composé (I) à partir d'un mélange éthanol-eau dans un rapport de 97 : 3 à 95 : 5 additionné d'éther diisopropylique, dans lequel le mélange éthanol-eau est concentré sous pression réduite à une température comprise entre 30 et 55°C jusqu'à un volume compris entre 1/2 et 1/3 du volume initial et l'addition de l'éther diisopropylique est réalisée en au moins 5 minutes.

2. Procédé selon la revendication 1 comprenant en outre l'étape de recristallisation à partir d'un solvant protique comprenant de l'éthanol, de l'isopropanol ou leurs mélanges aqueux.

3. Monochlorhydrate de 8-hydroxy-5-[(1R)-1-hydroxy-2-[[(1R)-2-(4-méthoxyphényl)-1-méthyléthyl]amino] éthyl]-2(1H)-quinolinone pouvant être obtenu par le procédé selon l'une quelconque des revendications 1 - 2 ayant un degré de cristallinité exprimé en % en poids du composé cristallin par rapport au poids total du composé d'au moins 93 %.

4. Monochlorhydrate de 8-hydroxy-5-[(1R)-1-hydroxy-2-[[(1R)-2-(4-méthoxyphényl)-1-méthyléthyl]amino] éthyl]-2(1H)-quinolinone selon la revendication 3 ayant un degré de cristallinité exprimé en % en poids du composé cristallin par rapport au poids total du composé d'au moins 95 %.

5. Monochlorhydrate de 8-hydroxy-5-[(1R)-1-hydroxy-2-[[(1R)-2-(4-méthoxyphényl)-1-méthyléthyl]amino] éthyl]-2(1H)-quinolinone selon l'une quelconque des revendications 3 - 4 ayant une plage de fusion de 185-195°C déterminée par calorimétrie différentielle à balayage.

6. Monochlorhydrate de 8-hydroxy-5-[(1R)-1-hydroxy-2-[[(1R)-2-(4-méthoxyphényl)-1-méthyléthyl]amino] éthyl]-2(1H)-quinolinone selon l'une quelconque des

revendications 3 - 4 ayant une plage de fusion de 190-195°C déterminée par calorimétrie différentielle à balayage.

Fig. 1: X-ray powder diffraction (XRD) pattern of the compound of Example 1.

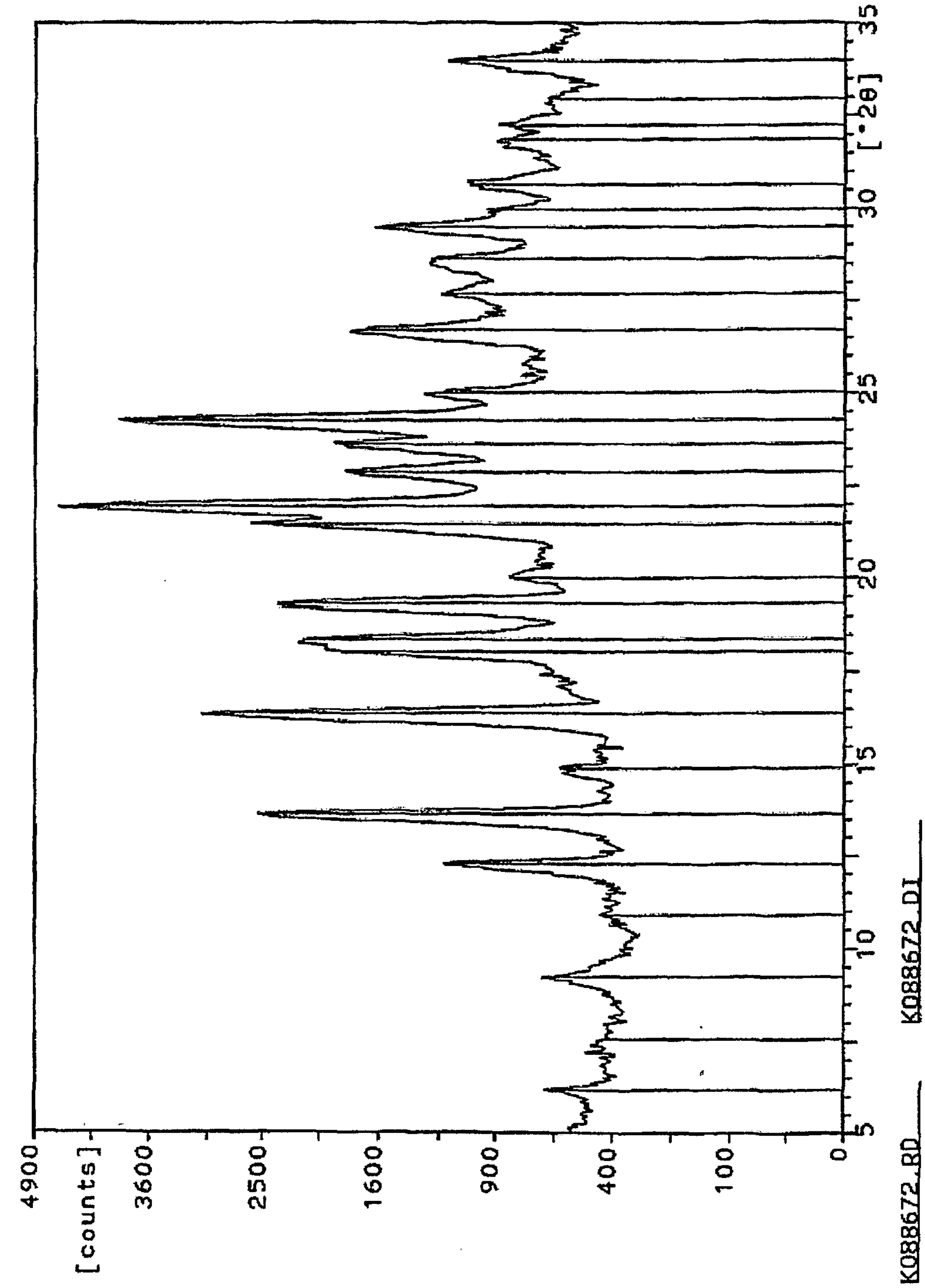

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0147719 A
- JP 9309830 A
- US 6030604 A
- WO 2005013945 A


### Non-patent literature cited in the description


- **KIKKAWA H et al.** *Japan J Pharmacol,* 1991, vol. 57, 175-185
- **VOSS HP et al.** *Eur J Pharmacol - Mol Pharmacol,* 1992, 403-409
- **IZEBOUD CA et al.** *Naunyn-Schmiedeberg's Archv Pharmacol,* 2000, vol. 362 (2), 184-189
- *J Pharmacol,* 2005, vol. 144, 422-429